# EUROPEAN PATENT APPLICATION

(11) **EP 2 422 704 A2**
(43) Date of publication of application: **29.02.2012**
(21) Application number: 11175026.1
(22) Date of filing: 22.07.2011
(51) Int. Cl.: A61B 8/00, G01S 15/89

(54) **Providing ultrasound spatial compound images in an ultrasound system**

(30) Priority: 31.08.2010 KR 20100084944
(71) Applicant: Samsung Medison Co., Ltd., Nam-myun Hongchun-gun Kangwon do 250-875 (KR)
(72) Inventor: Yoo, Jae Heung, 135-851 Seoul (KR)
(74) Representative: Schmid, Wolfgang

(57) **Abstract**

Embodiments for providing ultrasound spatial compound images are disclosed. In one embodiment, by way of non-limiting example, an ultrasound system comprises: an ultrasound data acquisition unit configured to acquire ultrasound data for a periodically moving target object; and a processing unit in communication with the ultrasound data acquisition unit, the processing unit being configured to form a plurality of ultrasound images based on the ultrasound data, detect moving periods of the target object based on the ultrasound images, reconstruct the ultrasound images corresponding to each of the moving periods and perform spatial compounding upon the reconstructed ultrasound images to form ultrasound spatial compound images.

## Description

### CROSS-REFERENCE TO RELATED APPLICATION

The present application claims priority from Korean Patent Application No. 10-2010-0084944 filed on August 31, 2010.

### TECHNICAL FIELD

The present disclosure generally relates to ultrasound systems, and more particularly to providing ultrasound spatial compound images in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two-dimensional or three-dimensional ultrasound images of internal features of a target object (e.g., human organs).

The ultrasound system transmits and receives ultrasound signals to and from a living body to thereby form a 2D (two-dimensional) ultrasound image or a 3D (three-dimensional) ultrasound image. Various techniques have been studied to enhance resolution of the ultrasound image. A spatial compounding is known as one of such techniques.

The spatial compounding is an imaging technique for forming a compound image by combining ultrasound images. That is, the ultrasound system forms a plurality of ultrasound images and performs the spatial compounding upon the ultrasound images to form an ultrasound spatial compound image.

However, the ultrasound spatial compound image may not be useful in certain cases, e.g., when observing a moving target object such as a heart, a fetus in the uterus, etc. in real time. That is, there is a problem in that an excessive blurring occurs in the ultrasound spatial compound image due to the motion of the target object.

### SUMMARY

Embodiments for providing ultrasound spatial compound images in an ultrasound system are disclosed herein. In one embodiment, by way of non-limiting example, an ultrasound system comprises: an ultrasound data acquisition unit configured to acquire ultrasound data for a periodically moving target object; and a processing unit in communication with the ultrasound data acquisition unit, the processing unit being configured to form a plurality of ultrasound images based on the ultrasound data, detect moving periods of the target object based on the ultrasound images, reconstruct the ultrasound images corresponding to each of the moving periods and perform spatial compounding upon the reconstructed ultrasound images to form ultrasound spatial compound images.

In another embodiment, there is provided a method of providing ultrasound spatial compound images, comprising: a) acquiring ultrasound data for a periodically moving target object; b) forming a plurality of ultrasound images based on the ultrasound data; c) detecting moving periods of the target object based on the ultrasound images; d) reconstructing the ultrasound images corresponding to each of the moving periods; and e) performing a spatial compounding upon the reconstructed ultrasound images to form ultrasound spatial compound images.

This summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a flow chart showing a process of forming ultrasound spatial compound images.
FIG. 4 is a schematic diagram showing an example of ultrasound images.
FIG. 5 is a schematic diagram showing an example of setting a feature point on an ultrasound image.
FIG. 6 is a schematic diagram showing an example of forming a feature point curve based on distances between principal axis and feature points.
FIG. 7 is a schematic diagram showing an example of the feature point curve.
FIG. 8 is a schematic diagram showing an example of forming ultrasound spatial compound images based on moving periods of a target object.

### DETAILED DESCRIPTION

A detailed description may be provided with reference to the accompanying drawings. One of ordinary skill in the art may realize that the following description is illustrative only and is not in any way limiting.

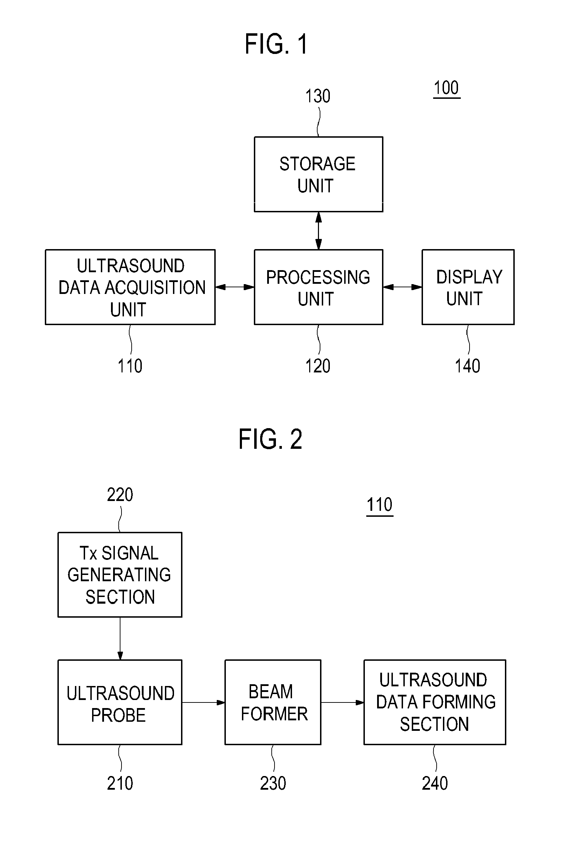

Referring to FIG. 1, an ultrasound system 100 in accordance with an illustrative embodiment is shown. As depicted therein, the ultrasound system 100 includes an ultrasound data acquisition unit 110. The ultrasound data acquisition unit 110 is configured to transmit and receive ultrasound signals to and from a living body and output ultrasound data. The living body includes a plurality of target objects (e.g., blood vessels, a heart, etc.).

FIG. 2 is a block diagram showing an illustrative embodiment of the ultrasound data acquisition unit 110. Referring to FIG. 2, the ultrasound data acquisition unit 110 includes an ultrasound probe 210. The ultrasound probe 210 includes a plurality of elements (not shown) for reciprocally converting between ultrasound signals and electrical signals. The ultrasound probe 210 is configured to transmit ultrasound signals to the living body. The ultrasound probe 210 is further configured to receive ultrasound signals (i.e., ultrasound echo signals) from the living body to output received signals. The received signals are analog signals. The ultrasound probe 210 includes a three-dimensional probe, a two-dimensional array probe, a convex probe and the like.

The ultrasound data acquisition unit 110 further includes a transmit (Tx) signal generating section 220. The Tx signal generating section 220 is configured to control the transmission of the ultrasound signals. The Tx signal generating section 220 is further configured to generate electrical signals ("Tx signals") for obtaining an ultrasound image in consideration of the elements and focal points. The ultrasound image includes a brightness mode image. However, it should be noted herein that the ultrasound image may not be limited thereto.

In one embodiment, the Tx signal generating section 220 is configured to generate Tx signals for obtaining a plurality of ultrasound images. Thus, the ultrasound probe 210 is configured to convert the Tx signals provided from the Tx signal generating section 220 into the ultrasound signals, transmit the ultrasound signals to the living body and receive the ultrasound echo signals from the living body to thereby output the received signals.

The ultrasound data acquisition unit 110 further includes a beam former 230. The beam former 230 is configured to convert the received signals provided from the ultrasound probe 210 into digital signals. The beam former 230 is further configured to apply delays to the digital signals in consideration of the elements and the focal points to thereby output digital receive-focused signals.

The ultrasound data acquisition unit 110 further includes an ultrasound data forming section 240. The ultrasound data forming section 240 is configured to form ultrasound data corresponding to each of the ultrasound images based on the digital receive-focused signals provided from the beam former 230. The ultrasound data includes radio frequency data. However, it should be noted herein that the ultrasound data may not be limited thereto. The ultrasound data forming section 240 is further configured to perform signal processing (e.g., gain control, etc) upon the digital receive-focused signals.

Although it is described above that the ultrasound data acquisition unit 110 is configured to acquire the ultrasound data by transmitting and receiving the ultrasound signals to and from the living body, the ultrasound data acquisition unit 110 is further configured to acquire the ultrasound data from an external or internal storage unit (not shown) connected to the ultrasound system 100.

Referring back to FIG. 1, the ultrasound system 100 further includes a processing unit 120 in communication with the ultrasound data acquisition unit 110. The processing unit 120 includes a central processing unit, a microprocessor or a graphic processing unit. However, it should be noted herein that the processing unit 120 may not be limited thereto.

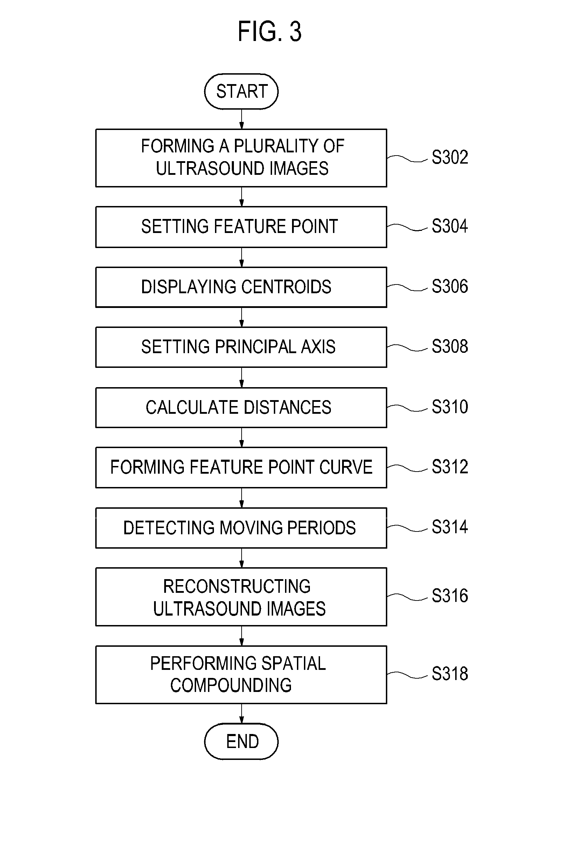

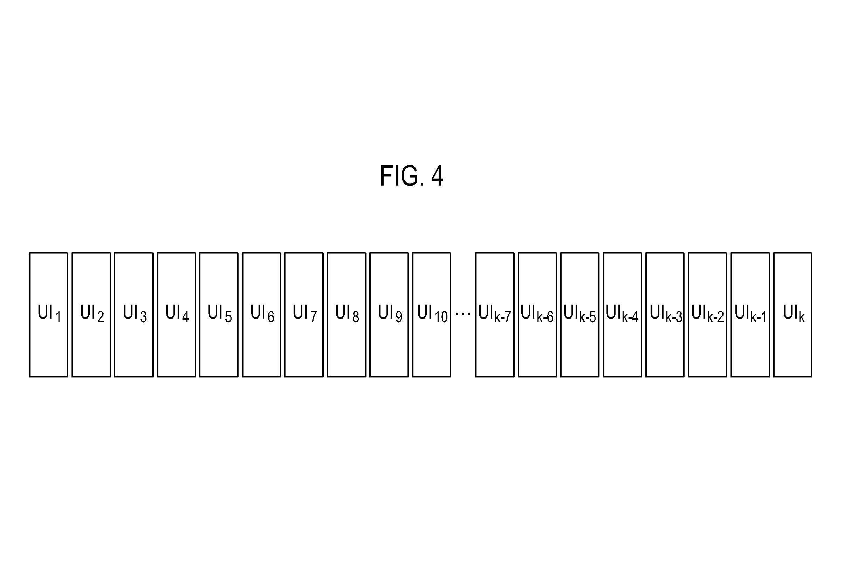

FIG. 3 is a flow chart showing a process of forming ultrasound spatial compound images. The processing unit 120 is configured to form a plurality of ultrasound images UI₁, UI₂ ... UI_{K} as shown in FIG. 4, based on the ultrasound data provided from the ultrasound data acquisition unit 110, at step S302 in FIG. 3.

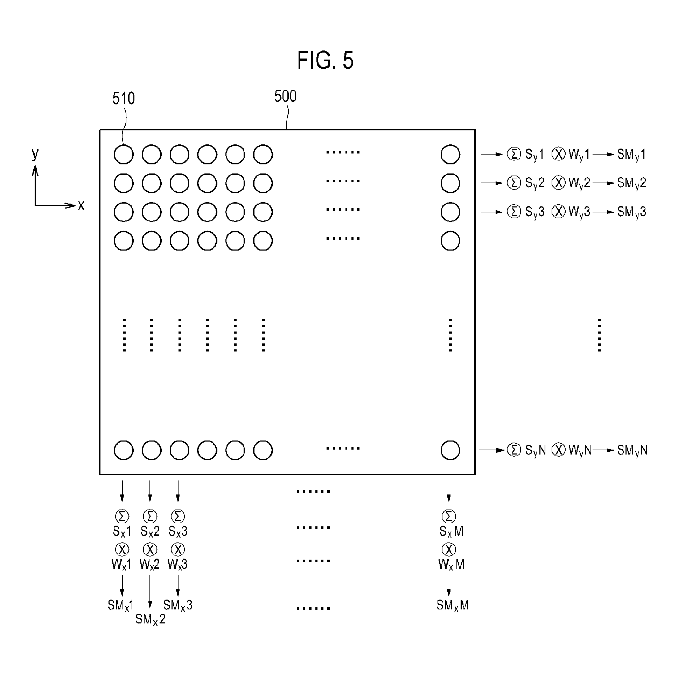

The processing unit 120 is configured to set a feature point on each of the ultrasound images at step S304 in FIG. 3. The feature point is set by using a common feature on each of the ultrasound images. In one embodiment, the feature point is set by using a centroid of brightness values constituting each of the ultrasound images. A method of determining the centroid of the brightness values will be described by using an ultrasound image 500, which has M x N pixels 510, as shown in FIG. 5.

FIG. 5 is a schematic diagram showing an example of setting the feature point on the ultrasound image 500. For the sake of convenience, it will be described that the ultrasound image 500 is placed on the X-Y plane of rectangular coordinate system in which the X coordinates of the ultrasound image 500 ranges from 1 to M and the Y coordinates of the ultrasound image 500 ranges from 1 to N. The processing unit 120 is configured to vertically sum the pixel values at each of the X coordinates 1 to M in the ultrasound image 500. That is, assuming that brightness values in the ultrasound image 500 are represented by P_{XY}, the processing unit 120 is configured to sum P_{X1}, P_{X2} ... P_{XN} to thereby output first sums Sx1-SxM corresponding to the respective X coordinates. Subsequently, the processing unit 120 is further configured to multiply the first sums Sx1-SxM by weights W_{X}1-WxM, respectively, to thereby output first weighted sums SMx1-SMxM. In one embodiment, the weights Wx1-WxM are determined by arbitrary values, which increase or decrease at a constant interval. For example, the numbers 1-M are used as the weight values Wx1-WxM. The processing unit 120 is further configured to sum all of the first sums Sx1-SxM to thereby output it as a second sum value, as well as sum all of the first weighted sums SMx1-SM to thereby output it as a third sum value. The processing unit 120 is further configured to divide the third sum value by the second sum value. The division result is set as the centroid on the X axis.

Also, the processing unit 120 is configured to horizontally sum the pixel values at each of the Y coordinates 1-N in the ultrasound image 500. That is, assuming that brightness values in the ultrasound image 500 are represented by P_{XY}, the processing unit 120 is configured to sum P_{1Y}, P_{2Y} ... P_{MY} to thereby output fourth sums Sy1-SyN corresponding to the respective Y coordinates. Subsequently, the processing unit 120 is further configured to multiply the fourth sums Sy1-SyN by weights Wy1-WyN, respectively, to thereby output second weighted sums SMy1-SMyN. In one embodiment, the weights Wy1-WyN are determined by arbitrary values, which increase or decrease at a constant interval. For example, the numbers 1-N are used as the weight values Wy1-WyN. The processing unit 120 is further configured to sum all of the fourth sums Sy1-SyN to thereby output a fifth sum, as well as sum all of the second weighted sums SMy1-SM to thereby output a sixth sum. The processing unit 120 is further configured to divide the sixth sum by the fifth sum and then set the division result as the centroid on the Y axis.

Although it is described that the feature point is set by using the centroid of brightness values constituting each of the ultrasound images, the feature point setting is certainly not limited thereto.

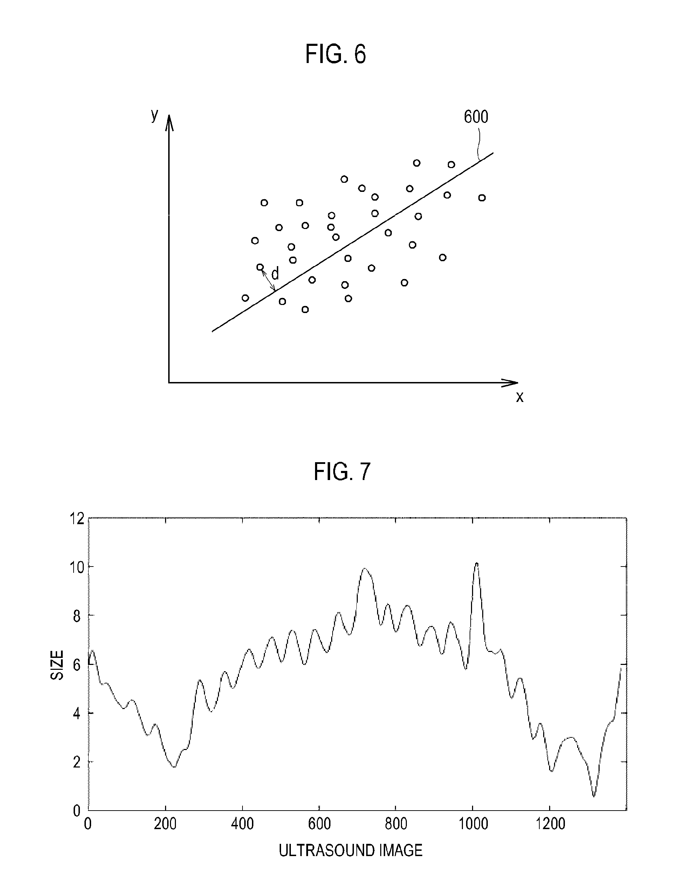

Once the setting of the centroid is complete for all of the ultrasound images, the processing unit 120 is configured to set centroids on the X-Y coordinate system, at step S306 in FIG. 3 and then set a principal axis 600 thereon as shown in FIG. 6, at step S308 in FIG. 3.

FIG. 6 is a schematic diagram showing an example of forming the feature point curve based on distances between the principal axis and feature points. The processing unit 120 is configured to calculate distances "d" from the principal axis 600 to each of the centroids, at step S310 in FIG. 3.

FIG. 7 is a schematic diagram showing an example of a feature point curve. The processing unit 120 is configured to form the feature point curve by using the computed distances as shown in FIG. 7, at step S312 in FIG. 3.

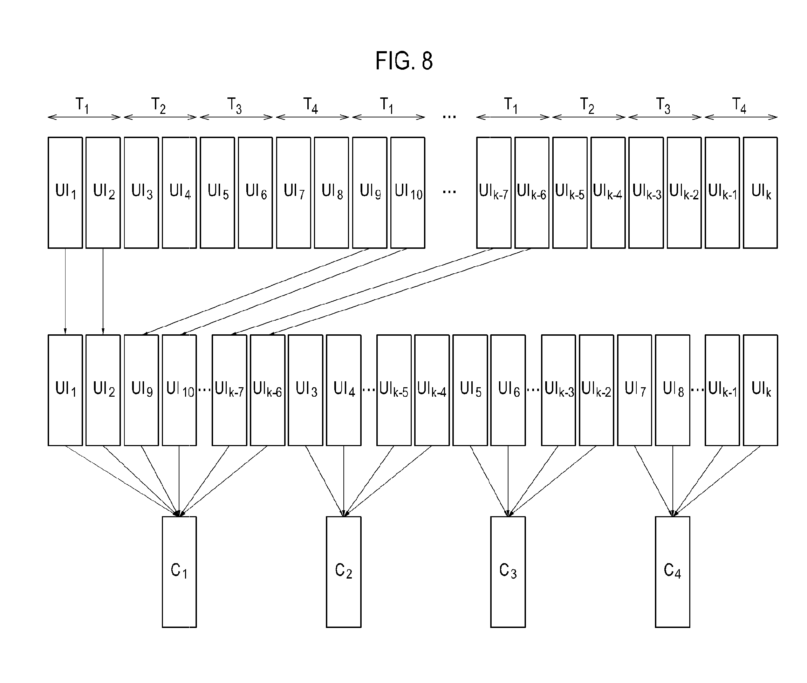

The processing unit 120 is configured to detect moving periods of the target object by using peak points in the graph shown in FIG. 7, at step S314 in FIG. 3. In one embodiment, the processing unit 120 is configured to calculate the gradients in the feature point curve in FIG. 7. The processing unit 120 is further configured to calculate zero cross points, the gradient of which changes from positive to negative, and then detect the zero cross points having a similar distance, to thereby detect periods of the detected zero cross points to the moving periods T_{1,} T₂, T₃ and T₄ of the target object as shown in FIG. 8.

The processing unit 120 is configured to reconstruct ultrasound images corresponding to each of the moving periods, at step S316 in FIG. 3. In one embodiment, the processing unit 120 is configured to reconstruct the ultrasound images UI₁, UI₂, UI₉, UI₁₀ ... UI_{K}-₇ and UI_{K-6} corresponding to the moving period T₁ from the ultrasound images UI₁ to UI_{K}, as shown in FIG. 8. The processing unit 120 is further configured to reconstruct the ultrasound images UI₃, UI₄ ... UI_{K}-₅ and UI_{K4} corresponding to the moving period T₂ from the ultrasound images UI₁ to UI_{K}, as shown in FIG. 8. The processing unit 120 is further configured to reconstruct the ultrasound images UI₅, UI₆ ... UI_{K}-₃ and UI_{K}-₂ corresponding to the moving period T₃ from the ultrasound images UI₁ to UI_{K}, as shown in FIG. 8. The processing unit 120 is further configured to reconstruct the ultrasound images UI₇, UI₈ ... UI_{K}-₁ and UI_{K} corresponding to the moving period T₄ from the ultrasound images UI₁ to UI_{K}, as shown in FIG. 8.

Further, while the ultrasound images are being acquired by scanning the living body, the target object (e.g., expectant mother or fetus) is moved. This makes it difficult to accurately detect the heartbeat of the fetus. Accordingly, the processing unit 120 is further configured to compensate the motion of the expectant mother or the fetus by matching the brightness of pixels. The detailed description relating to the method of compensating the motion is omitted herein since the conventional methods may be used.

The processing unit 120 is configured to perform spatial compounding upon the reconstructed ultrasound images corresponding to each of the moving periods to form ultrasound spatial compound images, at step S318 in FIG. 3. In one embodiment, the processing unit 120 is configured to perform the spatial compounding upon the reconstructed ultrasound images UI₁, UI₂, UI₉, UI₁₀ ... UI_{K}-₇ and UI_{K}-₆ corresponding to the moving period T₁ to form the ultrasound spatial compound image C₁ corresponding to the moving period T_{1,} as shown in FIG. 8. The processing unit 120 is further configured to perform the spatial compounding upon the reconstructed ultrasound images UI₃, UI₄ ... UI_{K}-₅ and UI_{K-4} corresponding to the moving period T₂ to form the ultrasound spatial compound image C₂ corresponding to the moving period T₂, as shown in Fig. 8. The processing unit 120 is further configured to perform the spatial compounding upon the reconstructed ultrasound images UI₅, UI₆ ... UI_{K}-₃ and UI_{K}-₂ corresponding to the moving period T₃ to form the ultrasound spatial compound image C₃ corresponding to the moving period T₃, as shown in FIG. 8. The processing unit 120 is further configured to perform the spatial compounding upon the reconstructed ultrasound images UI₇, UI₈ ... UI_{K-1} and UI_{K} corresponding to the moving period T₄ to form the ultrasound spatial compound image C₄ corresponding to the moving period T₄, as shown in FIG. 8.

Referring again to FIG. 1, the ultrasound system 100 further includes a storage unit 130. The storage unit 130 stores the ultrasound data acquired by the ultrasound data acquisition unit 110. The storage unit 130 further stores the ultrasound images formed by processing unit 120.

The ultrasound system 100 further includes a display unit 140. The display unit 140 is configured to display the ultrasound spatial compound images formed by the processing unit 120. The display unit 140 is further configured to display the ultrasound images formed by the processing unit 120.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to acquire ultrasound data for a periodically moving target object; and
a processing unit in communication with the ultrasound data acquisition unit,
the processing unit being configured to form a plurality of ultrasound images based on the ultrasound data, detect moving periods of the target object based on the ultrasound images, reconstruct the ultrasound images corresponding to each of the moving periods and perform spatial compounding upon the reconstructed ultrasound images to form ultrasound spatial compound images.

2. The ultrasound system of Claim 1, wherein the processing unit is configured to:
set a feature point on each of the ultrasound images based on brightness values of pixels included therein;
form a feature point curve based on the feature points; and
detect the moving periods of the target object based on the feature point curve.

3. The ultrasound system of Claim 2, wherein the processing unit is configured to set a centroid of the brightness values on each of the ultrasound images to the feature point.

4. The ultrasound system of Claim 2, wherein the processing unit is configured to:
set a principal axis based on positions of the feature points; and
form the feature point curve based on distances between the feature points and the principal axis.

5. The ultrasound system of Claim 2, wherein the processing unit is configured to:
calculate gradients from the feature point curve;
detect zero crossing points that a sign of the gradient changes from positive to negative; and
determine the moving period based on intervals between the detected zero crossing points.

6. The ultrasound system of Claim 2, wherein the processing unit is further configured to compensate the motion of the target object.

7. A method of providing ultrasound spatial compound images, comprising:
a) acquiring ultrasound data for a periodically moving target object;
b) forming a plurality of ultrasound images based on the ultrasound data;
c) detecting moving periods of the target object based on the ultrasound images;
d) reconstructing the ultrasound images corresponding to each of the moving periods; and
e) performing a spatial compounding upon the reconstructed ultrasound images to form ultrasound spatial compound images.

8. The method of Claim 7, wherein the step c) comprises:
c1) setting a feature point on each of the ultrasound images based on brightness values of pixels included therein;
c2) forming a feature point curve based on the feature points; and
c3) detecting the moving periods of the target object based on the feature point curve.

9. The method of Claim 8, wherein the step c1) comprises:
setting a centroid of the brightness values on each of the ultrasound images to the feature point.

10. The method of Claim 8, wherein the step c2) comprises:
setting a principal axis based on positions of the feature points; and
forming the feature point curve based on distances between the feature points and the principal axis.

11. The method of Claim 8, wherein the step c3) comprises:
calculating gradients from the feature point curve;
detecting zero crossing points that a sign of the gradient changes from positive to negative; and
determining the moving period based on intervals between the detected zero crossing points.

12. The method of Claim 8, wherein the step c) further comprises:
compensating the motion of the target object.
